# EUROPEAN PATENT APPLICATION

(11) **EP 4 317 182 A1**
(43) Date of publication of application: **07.02.2024**
(21) Application number: 22774186.5
(22) Date of filing: 21.03.2022
(51) Int. Cl.: C07K 16/12, C12N 15/13, A61K 39/40, A61P 31/04, G01N 33/68

(54) **ANTIGEN-BINDING PROTEIN TARGETING STREPTOCOCCUS PNEUMONIAE HEMOLYSIN PROTEIN AND USE THEREOF**

(30) Priority: 22.03.2021 CN 202110301731
(71) Applicant: STARMAB BIOLOGICS (SUZHOU) CO., LTD, Suzhou, Jiangsu 215101 (CN); Starmab Biologics (Shanghai) Co., Ltds, Shanghai 201908 (CN)
(72) Inventor: AN, Maomao, Suzhou, Jiangsu 215101 (CN); GUO, Shiyu, Suzhou, Jiangsu 215101 (CN); CHEN, Simin, Suzhou, Jiangsu 215101 (CN); QIU, Xiran, Suzhou, Jiangsu 215101 (CN); LI, Bohua, Suzhou, Jiangsu 215101 (CN)
(74) Representative: Arnold & Siedsma
(86) International application number: PCT/CN2022/082011
(87) International publication number: WO 2022/199527

(57) **Abstract**

Provided is an isolated antigen-binding protein, which comprises at least one CDR in an antibody heavy chain variable region VH, wherein the VH comprises the amino acid sequence as shown in SEQ ID NO: 39.The isolated antigen-binding protein can specifically bind to *Streptococcus pneumoniae* hemolysin protein and variants thereof. Further provided is the use of the isolated antigen-binding protein in prevention and/or treatment of diseases and/or conditions of a *Streptococcus pneumoniae* infection.

## Description

### Technical Field

This application relates to the field of biomedicine, in particular to an antigen binding protein targeting pneumolysin protein (PLY), and the use thereof in the treatment and prevention of *Streptococcus pneumoniae* infection.

### Background

*Streptococcus pneumoniae*, also known as pneumococcus, is widely colonized in the nasopharynx of humans. It can cause lung or central nervous system infections when resistance decrease, and in severe cases, it causes bacteremia or sepsis.

Pneumolysin (PLY) is an important virulence factor of *Streptococcus pneumoniae* and is important for the infection of *Streptococcus pneumoniae.* PLY can cause damage to lung tissue epithelium, endothelium and immune cells, increase vascular permeability, increase colonization, spread and invasion of *Streptococcus pneumoniae*, and affect the lung function of patients, resulting in acute lung injury and even acute respiratory distress syndrome. Therefore, it is urgent to develop effective drugs against *Streptococcus pneumoniae* infection.

### Summary

The present application provides an isolated antigen-binding protein, which has one or more of the following features: 1) it can specifically bind to pneumolysin protein and its variants; 2) it has high affinity and neutralizing activity to pneumolysin protein; 3) it can effectively block infection caused by *Streptococcus pneumoniae*; 4) it can effectively prevent and/or treat diseases and/or conditions caused by *Streptococcus pneumoniae.*

In one aspect, the present invention provides an isolated antigen-binding protein comprising at least one CDR in the heavy chain variable region VH of an antibody , the VH comprising the amino acid sequence shown in SEQ ID NO: 39.

In some embodiments, the isolated antigen binding protein contains HCDR3 that comprises an amino acid sequence shown in SEQ ID NO: 1.

In some embodiments, the isolated antigen binding protein contains HCDR2 that comprises an amino acid sequence shown in SEQ ID NO: 2.

In some embodiments, the isolated antigen binding protein contains HCDR1 that comprises an amino acid sequence shown in SEQ ID NO: 3.

In some embodiments, the isolated antigen binding protein comprises HCDR1, HCDR2 and HCDR3, the HCDR1 comprises an amino acid sequence shown in SEQ ID NO: 3, the HCDR2 comprises an amino acid sequence shown in SEQ ID NO: 2, and the HCDR3 comprises an amino acid sequence shown in SEQ ID NO: 1.

In some embodiments, the isolated antigen binding protein comprises H-FR1, the C terminus of H-FR1 is directly or indirectly linked to the N terminus of the HCDR1, and the H-FR1 comprises an amino acid sequence shown in SEQ ID NO: 32.

In some embodiments, the H-FR1 comprises an amino acid sequence shown in SEQ ID NO: 7 or SEQ ID NO: 15.

In some embodiments, the isolated antigen binding protein comprises H-FR2, the H-FR2 is located between the HCDR1 and HCDR2, and the H-FR2 comprises an amino acid sequence shown in SEQ ID NO: 33.

In some embodiments, the H-FR2 comprises an amino acid sequence shown in SEQ ID NO: 8 or SEQ ID NO: 16.

In some embodiments, the isolated antigen binding protein comprises H-FR3, the H-FR3 is located between the HCDR2 and HCDR3, and the H-FR3 comprises an amino acid sequence shown in SEQ ID NO: 34.

In some embodiments, the H-FR3 comprises an amino acid sequence shown in SEQ ID NO: 9 or SEQ ID NO: 17.

In some embodiments, the isolated antigen binding protein comprises H-FR4, the N terminus of which is directly or indirectly linked to the C terminus of the HCDR3, and the H-FR4 comprises an amino acid sequence shown in SEQ ID NO: 35.

In some embodiments, the H-FR4 comprises an amino acid sequence shown in SEQ ID NO: 10 or SEQ ID NO: 18.

In some embodiments, the isolated antigen binding protein comprises H-FR1, H-FR2, H-FR3 and H-FR4, the H-FR1 comprises an amino acid sequence shown in SEQ ID NO: 32, the H-FR2 comprises an amino acid sequence shown in SEQ ID NO: 33, the H-FR3 comprises an amino acid sequence shown in SEQ ID NO: 34, and the H-FR4 comprises an amino acid sequence shown in SEQ ID NO: 35.

In some embodiments, the isolated antigen binding protein comprises H-FR1, H-FR2, H-FR3 and H-FR4, the H-FR1 comprises an amino acid sequence shown in SEQ ID NO: 7 or SEQ ID NO: 15, the H-FR2 comprises an amino acid sequence shown in SEQ ID NO: 8 or SEQ ID NO: 16, the H-FR3 comprises an amino acid sequence shown in SEQ ID NO: 9 or SEQ ID NO: 17, and the H-FR4 comprises an amino acid sequence shown in SEQ ID NO: 10 or SEQ ID NO: 18.

In some embodiments, the isolated antigen binding protein comprises H-FR1, H-FR2, H-FR3, and H-FR4 selected from any of the following groups:
1) the H-FR1 comprises an amino acid sequence shown in SEQ ID NO: 7, the H-FR2 comprises an amino acid sequence shown in SEQ ID NO: 8, the H-FR3 comprises an amino acid sequence shown in SEQ ID NO: 9, and the H-FR4 comprises an amino acid sequence shown in SEQ ID NO: 10; and
2) the H-FR1 comprises an amino acid sequence shown in SEQ ID NO: 15, the H-FR2 comprises an amino acid sequence shown in SEQ ID NO: 16, the H-FR3 comprises an amino acid sequence shown in SEQ ID NO: 17, and the H-FR4 comprises an amino acid sequence shown in SEQ ID NO: 18.

In some embodiments, the isolated antigen binding protein contains VH that comprises an amino acid sequence shown in SEQ ID NO: 39.

In some embodiments, the isolated antigen binding protein comprises VH, which comprises an amino acid sequence shown in SEQ ID NO: 22 or SEQ ID NO: 24.

In some embodiments, the isolated antigen binding protein comprises a heavy chain constant region of an antibody.

In some embodiments, the heavy chain constant region of the isolated antigen binding protein derived from human IgG constant region.

In some embodiments, the heavy chain constant region of the isolated antigen binding protein derived from human IgG1 heavy constant region.

In some embodiments, the heavy constant region of the isolated antigen binding protein contains an amino acid sequence shown in SEQ ID NO: 26.

In some embodiments, the isolated antigen binding protein contains heavy chain that comprises an amino acid sequence shown in SEQ ID NO: 28 or SEQ ID NO: 30.

In some embodiments, the isolated antigen binding protein comprises at least one CDR in the light chain variable region VL of an antibody, the VL comprises an amino acid sequence shown in SEQ ID NO: 40.

In some embodiments, the isolated antigen binding protein contains LCDR3 that comprises an amino acid sequence shown in SEQ ID NO: 4.

In some embodiments, the isolated antigen binding protein contains LCDR2 that comprises an amino acid sequence shown in SEQ ID NO: 5.

In some embodiments, the isolated antigen binding protein contains LCDR1 that comprises an amino acid sequence shown in SEQ ID NO: 6.

In some embodiments, the isolated antigen binding protein comprises LCDR1, LCDR2 and LCDR3, the LCDR1 comprises an amino acid sequence shown in SEQ ID NO: 6, the LCDR2 comprises an amino acid sequence shown in SEQ ID NO: 5, and the LCDR3 comprises an amino acid sequence shown in SEQ ID NO: 4.

In some embodiments, the isolated antigen binding protein comprises L-FR1, the C terminus of which is directly or indirectly linked to the N terminus of the LCDR1, and the L-FR1 comprises an amino acid sequence shown in SEQ ID NO: 36.

In some embodiments, the L-FR1 of the isolated antigen binding protein contains an amino acid sequence shown in SEQ ID NO: 19.

In some embodiments, the isolated antigen binding protein comprises L-FR2, the L-FR2 is located between the LCDR1 and LCDR2, and the L-FR2 comprises an amino acid sequence shown in SEQ ID NO: 37.

In some embodiments, the L-FR2 of the isolated antigen binding protein contains an amino acid sequence shown in SEQ ID NO: 12 or SEQ ID NO: 20.

In some embodiments, the isolated antigen binding protein comprises L-FR3, the L-FR3 is located between the LCDR2 and LCDR3, and the L-FR3 comprises an amino acid sequence shown in SEQ ID NO: 38.

In some embodiments, the L-FR3 of the isolated antigen binding protein contains an amino acid sequence shown in SEQ ID NO: 13 or SEQ ID NO: 21.

In some embodiments, the isolated antigen binding protein comprises L-FR4, the N terminus of which is directly or indirectly linked to the C terminus of the LCDR3, and the L-FR4 comprises an amino acid sequence shown in SEQ ID NO: 14.

In some embodiments, the isolated antigen binding protein comprises L-FR1, L-FR2, L-FR3 and L-FR4, the L-FR1 comprises an amino acid sequence shown in SEQ ID NO: 36, the L-FR2 comprises an amino acid sequence shown in SEQ ID NO: 37, the L-FR3 comprises an amino acid sequence shown in SEQ ID NO: 38, and the L-FR4 comprises an amino acid sequence shown in SEQ ID NO: 14.

In some embodiments, the isolated antigen binding protein comprises L-FR1, L-FR2, L-FR3 and L-FR4, the L-FR1 comprises an amino acid sequence shown in SEQ ID NO: 11 or SEQ ID NO: 19, the L-FR2 comprises an amino acid sequence shown in SEQ ID NO: 12 or SEQ ID NO: 20, the L-FR3 comprises an amino acid sequence shown in SEQ ID NO: 13 or SEQ ID NO: 21, and the L-FR4 comprises an amino acid sequence shown in SEQ ID NO: 14.

In some embodiments, the isolated antigen binding protein comprises L-FR1, L-FR2, L-FR3, and L-FR4 selected from any of the following groups:
1) the L-FR1 comprises an amino acid sequence shown in SEQ ID NO: 11, the L-FR2 comprises an amino acid sequence shown in SEQ ID NO: 12, the L-FR3 comprises an amino acid sequence shown in SEQ ID NO: 13, and the L-FR4 comprises an amino acid sequence shown in SEQ ID NO: 14; and
2) the L-FR1 comprises an amino acid sequence shown in SEQ ID NO: 19, the L-FR2 comprises an amino acid sequence shown in SEQ ID NO: 20, the L-FR3 comprises an amino acid sequence shown in SEQ ID NO: 21, and the L-FR4 comprises an amino acid sequence shown in SEQ ID NO: 14.

In some embodiments, the isolated antigen binding protein contains VL that comprises an amino acid sequence shown in SEQ ID NO: 40.

In some embodiments, the VL of the isolated antigen binding protein contains an amino acid sequence shown in SEQ ID NO: 23 or SEQ ID NO: 25.

In some embodiments, the isolated antigen binding protein comprises a light chain constant region of an antibody.

In some embodiments, the light chain constant region of the isolated antigen binding protein derives from human IgG κ constant region.

In some embodiments, the light constant region of the isolated antigen binding protein contains an amino acid sequence shown in SEQ ID NO: 27.

In some embodiments, the isolated antigen binding protein contains light chain that comprises an amino acid sequence shown in SEQ ID NO: 29 or SEQ ID NO: 31.

In some embodiments, the isolated antigen binding protein comprises VH and VL selected from any of the following groups:
1) the VH comprises an amino acid sequence shown in SEQ ID NO: 22, and the VL comprises an amino acid sequence shown in SEQ ID NO: 23; and
2) the VH comprises an amino acid sequence shown in SEQ ID NO: 24, and the VL comprises an amino acid sequence shown in SEQ ID NO: 25.

In some embodiments, the isolated antigen binding protein includes an antibody or an antigen binding fragment thereof.

In some embodiments, the antigen binding fragment includes Fab, Fab', Fv fragment, F(ab')2, F(ab)2, scFv, di-scFv and/or dAb.

In some embodiments, the antibody is selected from one or more of the following groups: monoclonal antibody, chimeric antibody, humanized antibody, and fully human antibody.

In some embodiments, the isolated antigen binding protein can specifically bind to pneumolysin protein.

In some embodiments, the pneumolysin comprises wild-type pneumolysin protein and variants thereof.

In some embodiments, the pneumolysin protein comprises a pneumolysin protein variant in which amino acid residue 146 is deleted compared with the wild-type pneumolysin protein.

In some embodiments, the isolated antigen binding protein can prevent and/or treat diseases and/or conditions caused by *Streptococcus pneumoniae.*

In some embodiments, the diseases and/or conditions include complications of diseases and/or conditions caused by *Streptococcus pneumoniae.*

In another aspect, the present application also provides a polypeptide molecule comprising the isolated antigen binding protein.

In some embodiments, the polypeptide molecule comprises a fusion protein.

In another aspect, the present application also provides an immunoconjugate comprising the isolated antigen binding protein.

In another aspect, the present application also provides a nucleic acid molecule, which encodes the isolated antigen binding protein or the polypeptide molecule.

In another aspect, the present application also provides a vector comprising the nucleic acid molecule.

In another aspect, the present application also provides a cell comprising the nucleic acid molecule or the vector.

In another aspect, the present application also provides a pharmaceutical composition comprising the isolated antigen binding protein, the polypeptide molecule, the immunoconjugate, the nucleic acid molecule, the vector and/or the cell, and optionally a pharmaceutically acceptable carrier.

In another aspect, the present application also provides a method for preparing the isolated antigen binding protein, which comprises culturing the cell under the condition that the antigen binding protein is expressed.

In another aspect, the isolated antigen binding protein, the polypeptide molecule, the immunoconjugate, the nucleic acid molecule, the vector, the cell and/or the pharmaceutical composition described herein are used alone or in combination with other drugs.

In another aspect, the present application also provides a use of the isolated antigen binding protein, the polypeptide molecule, the immunoconjugate, the nucleic acid molecule, the vector, the cell and/or the pharmaceutical composition in the manufacture of a medicament for the prevention and/or treatment of diseases and/or conditions.

In some embodiments, the disease and/or condition and complication thereof is caused or mediated by *Streptococcus pneumoniae.*

In some embodiments, the diseases and/or conditions include complications of diseases and/or conditions caused or mediated by *Streptococcus pneumoniae.*

In another aspect, the present application also provides a method for detecting pneumolysin protein in a sample, wherein the method comprises using the isolated antigen binding protein, the polypeptide molecule, the immunoconjugate, the nucleic acid molecule, the vector, the cell and/or the pharmaceutical composition.

In another aspect, the present application also provides a kit for detecting pneumolysin protein in a sample, wherein the kit comprises the isolated antigen binding protein, the polypeptide molecule, the immunoconjugate, the nucleic acid molecule, the vector, the cell and/or the pharmaceutical composition.

In another aspect, the present application also provides use of the isolated antigen binding protein, the polypeptide molecule, the immunoconjugate, the nucleic acid molecule, the vector, the cell and/or the pharmaceutical composition in the manufacture of a kit for detecting the presence and/or content of pneumolysin protein in a sample.

Those skilled in the art can easily perceive other aspects and advantages of the present application from the following detailed description. Only exemplary embodiments of the present application are shown and described in the following detailed description. As those skilled in the art will recognize, the content of the present application enables those skilled in the art to make changes to the disclosed specific embodiments without departing from the spirit and scope of the invention involved in the present application. Accordingly, the descriptions in the drawings and specification of the present application are merely exemplary and not restrictive.

### Description of Figures

The specific features of the invention involved in the present application are shown in the appended claims. The features and advantages of the present invention to which this application relates can be better understood with reference to the exemplary embodiments described in detail hereinafter and the accompanying drawings. A brief description of the attached figures is as follows:
Figure 1 shows the assay of the binding activity of the antigen-binding protein described in the present application to PLY protein.
Figure 2 shows the assay of the binding activity of the antigen-binding protein described in the present application to PLYmut protein.
Figure 3 shows that antigen binding protein of the present application neutralizes the hemolytic activity of Pneumolysin destroying rabbit red blood cells *in vitro.*
Figure 4 shows the protection of the antigen-binding protein described in the present application on PLY toxin model mice.
Figure 5 shows the protection of the antigen-binding protein described in the present application on mice infected with *Streptococcus pneumoniae.*

### Detailed Description

The following is a description of the embodiment of the invention according to the present application by specific examples. Those skilled in the art can easily understand other advantages and effects of the invention according to the contents disclosed in the specification.

### Definition of Terms

In this application, the term "*Streptococcus pneumoniae* hemolysin" is used interchangeably with "Pneumolysin", "PLY" and "pneumolysin protein" and generally refers to a multifunctional virulence factor contained in *Streptococcus pneumoniae.* In the present application, the term may encompass wild-type pneumolysin proteins and variants, analogs, homologues and functionally active fragments thereof. For example, the variant of the pneumolysin protein may comprise a mutation that deletes amino acid residue 146 as compared to wild-type pneumolysin. For example, the functionally active fragment may be a moiety capable of eliciting a humoral and/or cellular immune response in a host.

In the present application, the term "isolated" usually refers to those obtained by artificial means from a natural state. If an "isolated" substance or component appears in nature, its natural environment may have been changed, or the substance may be separated from the natural environment, or both. For example, a living animal naturally has a certain kind of undissociated polynucleotide or polypeptide, and the same polynucleotide or polypeptide with high purity isolated from this natural state is called isolated. The term "isolated" does not exclude the mixing of artificial or synthetic substances, nor does it exclude the presence of other impure substances that do not affect the activity of the substance.

In the present application, the term "isolated antigen binding protein" generally refers to a protein with antigen binding ability that is separated from its naturally occurring state. The "isolated antigen binding protein" may comprise a portion that binds antigen, and optionally a framework or framework portion that allows the antigen binding portion to adopt the conformation that facilitates the antigen binding portion to bind to the antigen. The antigen binding protein may comprise, for example, antibody derived protein framework regions (FRs) or alternative protein framework regions or artificial framework regions with transplanted CDRs or CDR derivatives. Such frameworks include, but are not limited to, antibody derived framework regions containing mutations introduced, for example, to stabilize the three-dimensional structure of the antigen binding protein and fully synthesized framework regions containing, for example, biocompatible polymers. See, for example, Korndorfer et. al., 2003, Proteins: Structure, Function, and Bioinformatics, 53(1):121-129(2003); Roque et. al., Biotechnol. Prog. 20:639-654 (2004). Examples of the antigen binding protein include, but are not limited to: human antibody; humanized antibody; chimeric antibody; recombinant antibody; single chain antibody; bifunctional antibody; trifunctional antibody; tetrafunctional antibody; Fab, Fab', Fv fragment, Bs-Fv, F(ab')2, F(ab)2, scFv, di-scFv, dAb, IgD antibody; IgE antibody; IgM antibody; IgG1 antibody; IgG2 antibody; IgG3 antibody; or IgG4 antibody and fragments thereof.

In the present application, the term "CDR", also known as "complementarity determining region", usually refers to a region in the variable domain of an antibody whose sequence is highly variable and/or forms a structure defining loop. Typically, antibodies include six CDRs; three in VH (HCDR1, HCDR2, HCDR3), and three in VL (LCDR1, LCDR2, LCDR3).In some embodiments, naturally occurring camel antibodies composed of only heavy chains can also function normally and stably in the absence of light chains. See, for example, Hamers-Casterman et. al., Nature 363:446-448 (1993); Sheriff et. al., Nature Struct. Biol. 3:733-736 (1996).Antibody CDRs can be determined by a variety of coding systems, such as CCG, Kabat, AbM, Chothia, IMGT, combined Kabat/Chothia, etc. These coding systems are known in the art and can be found in, for example, http://www.bioinf.org.uk/abs/index.html#kabatnum.For example, the amino acid sequence of the antigen binding protein can be numbered according to the IMGT (the international ImMunoGeneTics information system@imgt.cines.fr; http: //imgt.cines.fr; http://imgt.cines.fr; Lefranc et. al., 1999, Nucleic Acids Res. 27: 209-212; Ruiz et. al.,2000 Nucleic Acids Res. 28: 219-221; Lefranc et. al., 2001, Nucleic Acids Res. 29:207-209; Lefranc et. al., 2003, Nucleic Acids Res. 31: 307-310; Lefranc et. al., 2005, DevComp Immunol 29: 185-203).For example, the CDRs of the antigen binding protein can be determined according to the Kabat numbering system (see, for example, Kabat EA &Wu TT (1971) Ann NY AcadSci 190:382-391 and Kabat EA et. al., (1991) Sequences of Proteins of Immunological Interest, FifthEdition, U.S. Department of Health and Human Services, NIH Publication No.91-3242).

In the present application, the term "FR" generally refers to a more highly conserved part of the antibody variable domain, which is referred to as the framework region. In general, the variable domains of natural heavy and light chains each contain four FR regions, namely four in VH (H-FR1, H-FR2, H-FR3, and H-FR4), and four in VL (L-FR1, L-FR2, L-FR3, and L-FR4).

In the present application, the terms "variable domain" and "variable region" can be used interchangeably, usually referring to a part of the antibody heavy chain and/or light chain. The variable domains of the heavy and light chains can be referred to as "V_{H}" and "V_{L}" (or "VH" and "VL", respectively). These domains are usually the most variable parts of antibodies (relative to other antibodies of the same type) and contain antigen binding sites.

In the present application, the term "variable" generally refers to that there may be large differences in the sequence of some segments of the variable domain between antibodies. Variable domains mediate antigen binding and determine the specificity of a specific antibody to its specific antigen. However, the variability is not evenly distributed throughout the variable domain. It is usually concentrated in three segments called hypervariable regions (CDRs or HVRs) in the light and heavy chain variable domains. The more highly conserved part of the variable domain is called the framework region (FR). The variable domains of natural heavy and light chains each contain four FR regions, most of which are in β-sheet configuration, connected by three CDRs, which form a ring linkage, and in some cases a part of a β-sheet structured in each chain are held together closely by the FR regions, and CDRs from the other chain together promote the formation of antigen binding sites of antibodies (see Kabat et. al., Sequences of Immunological Interest, Fifth Edition, National Institute of Health, Bethesda, Md. (1991)).

In the present application, the term "antibody" generally refers to immunoglobulin or fragments thereof or derivatives thereof, covering any polypeptide including an antigen binding site, no matter whether it is produced in vitro or in vivo. The term includes but is not limited to polyclonal, monoclonal, monospecific, multispecific, nonspecific, humanized, single chain, chimeric, synthetic, recombinant, hybrid, mutant and transplanted antibodies. Unless otherwise modified by the term "complete", such as in "complete antibody", for the purpose of the present invention, the term "antibody" also includes antibody fragments, such as Fab, F(ab')₂, Fv, scFv, Fd, dAb, and other antibody fragments that maintain antigen binding function (for example, specifically bind to pneumolysin protein). Generally, such fragments should include an antigen binding domain. The basic 4-chain antibody unit is a heterotetrameric glycoprotein composed of two identical light chains (L) and two identical heavy chains (H). IgM antibody consists of 5 basic heterotetramer units and another polypeptide called J chain, which contains 10 antigen binding sites; IgA antibody contains 2-5 basic 4 chain units, which can polymerize with J chain to form a multivalent assemblage. In the case of IgGs, the 4-chain unit is typically about 150,000 Daltons. Each light chain is connected to a heavy chain through a covalent disulfide bond, while the two heavy chains are connected to each other through one or more disulfide bonds, and the number of disulfide bonds depends on the isotype of the heavy chain. Each heavy and light chain also has an interchain disulfide bridge with regular spacing. Each heavy chain has a variable domain (VH) at the N-terminus, followed by three (for each α and γ chain) and four (for µ and ε isoforms) constant domains (CH).Each light chain has a variable domain (VL) at the N-terminus, followed by a constant domain (CL) at the other end. VL is aligned with VH, while CL is aligned with the first constant domain (CH1) of the heavy chain. Specific amino acid residues are thought to form an interface between light and heavy chain variable domains. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6.The L chain from any vertebrate species can be assigned to one of two clearly distinct types, called kappa and lambda, based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α, δ, ε, γ and µ, respectively.

In the present application, the term "antigen binding fragment" generally refers to one or more fragments with the ability to specific bind to an antigen (e.g., pneumolysin).In the present application, the antigen binding fragment may include Fab, Fab', F(ab)2, Fv fragment, F(ab')2, scFv, di-scFv and/or dAb.

In the present application, the term "Fab" generally refers to the antigen binding fragment of an antibody. As mentioned above, papain can be used to digest intact antibodies. The antibody digested by papain produces two identical antigen binding fragments, namely, "Fab" fragment and residual "Fc" fragment (namely Fc region, ibid.).The Fab fragment may consist of a complete L chain, a variable region of a heavy chain, and the first constant region (CH1) of the H chain (VH).

In the present application, the term " Fab' fragment" usually refers to the monovalent antigen binding fragment of a human monoclonal antibody, which is slightly larger than the Fab fragment. For example, Fab' fragments may include all light chains, all heavy chain variable regions, and all or part of the first and second constant regions of the heavy chain. For example, the Fab' fragment may also include some or all of the 220-330 amino acid residues of the heavy chain.

In the present application, the term "F(ab')2" generally refers to the antibody fragment generated by pepsin digestion of the complete antibody. The F(ab')2 fragment contains two Fab fragments and part of the hinge region maintained together by disulfide bonds. The F(ab')2 fragment has bivalent antigen binding activity and can cross link antigens.

In the present application, the term "Fv fragment" generally refers to the monovalent antigen binding fragment of a human monoclonal antibody, including all or part of the heavy chain variable region and light chain variable region, and lacks the heavy chain constant region and light chain constant region. The heavy chain variable region and the light chain variable region include, for example, Cursor example, Fv fragments include all or part of the amino terminal variable regions of about 110 amino acids of the heavy and light chains.

In the present application, the term "scFv" generally refers to a fusion protein comprising at least one antibody fragment including the variable region of the light chain and at least one antibody fragment including the variable region of the heavy chain, wherein the variable region of the light chain and the heavy chain are adjacent (for example, via a synthetic linker such as a short flexible polypeptide linker), and can be expressed as a single chain polypeptide, and wherein the scFv retains the specificity of the intact antibody from which it is derived. Unless otherwise specified, as used in the present application, scFv can have the VL and VH variable regions in any order (such as relative to the N-terminus and C-terminus of the polypeptide), and scFv can include VL-linker-VH or VH-linker-VL.

In the present application, the term "dAb" generally refers to an antigen binding fragment with a VH domain, a VL domain, or a VH domain or a VL domain, with reference to, for example, Ward et. al. (Nature, 1989Oct 12; 341 (6242):544-6), Holt et. al., Trends Biotechnol, 2003,21 (11):484-490; and referring to WO 06/030220, WO 06/003388 and other published patent applications by DomantisLtd. The term "dAb" generally includes sdAb. The term "sdAb" usually refers to a single domain antibody. Single domain antibodies usually refer to antibody fragments that consist only of the variable region of the antibody heavy chain (VH domain) or the variable region of the antibody light chain (VL).

In the present application, the term "monoclonal antibody" generally refers to an antibody molecule preparation consisting of a single molecule. Monoclonal antibodies are usually highly specific for a single antigen site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made in hybridoma cells or by recombinant DNA method.

In the present application, the term "chimeric antibody" generally refers to an antibody in which the variable region is derived from one species and the constant region is derived from another species. In general, the variable region is derived from antibodies of experimental animals such as rodents ("parental antibodies"), and the constant region is derived from human antibodies, causing the resulting chimeric antibodies less likely to elicit adverse immune reactions in human individuals than parental antibodies.

In the present application, the term "humanized antibody" generally refers to an antibody whose part or all of the amino acids of a non-human antibody other than the CDR region are replaced by the corresponding amino acids derived from a human immunoglobulin. In the CDR region, small additions, deletions, insertions, substitutions or modifications of amino acids can also be allowed, as long as they still retain the ability of antibodies to bind specific antigens. The humanized antibody may optionally comprise at least a portion of the constant region of human immunoglobulin. A "humanized antibody" retains antigen specificity similar to the original antibody. "Humanized" forms of non-human antibodies are chimeric antibodies that can contain a sequence derived from non-human immunoglobulin to the minimum. In one embodiment, a humanized antibody is a human immunoglobulin (recipient antibody) in which residues from an CDR of the recipient are replaced by residues from an CDR of a non-human species (donor antibody) such as alpaca, mouse, rat, rabbit or non-human primate having the desired specificity, affinity, and/or capacity. In some instances, framework ("FR") residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity.

The term "fully human antibody" generally refers to an antibody that contains only the human immunoglobulin protein sequence. If it is produced in mice, in mouse cells, or in hybridomas derived from mouse cells, the fully human antibody may contain murine glycans. Similarly, "mouse antibody" or "rat antibody" refer to antibodies containing only mouse or rat immunoglobulin sequences, respectively. Fully human antibody can be generated in human body and transgenic animals with human immunoglobulin germline sequence by phage display or other molecular biological methods. Exemplary techniques that can be used in the manufacture of antibodies are described in US patents: 6,150,584, 6,458,592, 6,420,140.Other techniques, such as the use of libraries, are known in the art.

In the present application, the terms "polypeptide molecule", "polypeptide" and "peptide" can be used interchangeably, usually referring to polymers of amino acid residues. The term "fusion protein" generally refers to a polypeptide with at least two covalently linked moieties. Each part can be a polypeptide with different properties. This property can be a biological property, such as in vitro or in vivo activity. This property can also be a simple chemical or physical property, such as binding to target molecules, catalysis of reactions, etc.These two parts can be connected through a single peptide bond or through a peptide linker.

In the present application, the term "nucleic acid molecule" generally refers to a nucleotide, deoxyribonucleotide or ribonucleotide in isolated form of any length, or an analog isolated from its natural environment or synthesized artificially.

In the present application, the term "vector" generally refers to a nucleic acid delivery vehicle that can insert a polynucleotide encoding a protein into it and enable the protein to be expressed. The vector can be transformed, transduced or transfected into host cells, so that the genetic material elements carried by it can be expressed in host cells. For example, vectors may include: plasmids; phagemid; cosmid; artificial chromosomes such as yeast artificial chromosome (YAC), bacterial artificial chromosome (BAC) or P1 derived artificial chromosome (PAC); phages such as λ Phage or M13 Phage, and animal virus, etc. Animal viruses used as vectors can include retrovirus (including lentivirus), adenovirus, adeno-associated virus, herpesvirus (such as herpes simplex virus), poxvirus, baculovirus, papillomavirus, papillomavacuolating virus (such as SV40).A vector may contain a variety of elements for controlling expression, including promoter sequence, transcription initiation sequence, enhancer sequence, selection element and reporter gene. In addition, the vector may also contain a replication start site. The vector may also include components that help it enter the cell, such as but not limited to viral particles, liposomes or protein coats.

In the present application, the term "cell" generally refers to a single cell, cell line or cell culture that can be or has been the recipient of the subject plasmid or vector, which includes the nucleic acid molecule or the vector described herein. Cells can include the progeny of a single cell. Due to natural, accidental or intentional mutations, the progeny may not necessarily be identical to the original parent cell (in the morphology of total DNA complement or in the genome).Cells may include cells transfected in vitro with the vector described herein. Cells can be bacterial cells (e.g., Escherichia coli), yeast cells, or other eukaryotic cells, such as COS cells, Chinese hamster ovary (CHO) cells, CHO-K1 cells, LNCAP cells, HeLa cells, HEK293 cells, COS-1 cells, NS0 cells.

In the present application, the term "immunoconjugate" generally refers to the conjugate formed by the conjugation of the other reagents (e.g., chemotherapeutic agents, radioactive elements, cell growth inhibitors, and cytotoxic agents) with the antibody or its antigen binding fragment (e.g., covalently linked by a linker molecule), which can specifically bind to the antigen on the target cell through the antibody or its antigen binding fragment to deliver the other reagents to target cells.

In the present application, the term "pharmaceutical composition" typically refers to a composition used for the prevention/treatment of diseases or conditions. The pharmaceutical composition may include isolated antigen binding proteins, nucleic acid molecules, vectors, and/or cells as described herein, and any pharmaceutically acceptable adjuvants. In addition, the pharmaceutical composition may also include one or more (pharmaceutically effective) vehicles, stabilizers, excipients, diluents, solubilizers, surfactants, emulsifiers, and/or preservatives. The acceptable components of the composition are preferably non-toxic to the recipient at the dosage and concentration used. The pharmaceutical compositions of the present invention include but are not limited to liquid, frozen, and lyophilized compositions.

In the present application, the term "pharmaceutically acceptable vehicle" usually includes pharmaceutically acceptable vehicles, excipients or stabilizers, which are non-toxic to cells or mammals exposed to them at the dose and concentration used. Physiologically acceptable carriers may include, for example, buffering agents, antioxidants, low molecular weight (less than about 10 residues) polypeptides, proteins, hydrophilic polymers, amino acids, monosaccharides, disaccharides, and other carbohydrates, chelating agents, sugar alcohols, salt forming counter ions, such as sodium, and/or non-ionic surfactants.

In the present application, the term "specific binding" or "specific" typically refer to measurable and reproducible interactions, such as the binding between a target and an antibody, which can determine the presence of a target in a heterogeneous population of molecules (including biomolecules).For example, antibodies that specifically bind to a target (which can be an epitope) can be antibodies that bind to that target with greater affinity, avidity, ease, and/or longer duration than other targets. In some embodiments, antibodies specifically bind to epitopes on proteins, which are conserved in proteins of different species. In certain embodiments, specific binding may include but not require exclusive binding. The term also includes, e.g., where an antigen binding protein is specific for a particular epitope that cross-reacts with multiple antigens, wherein a specific antibody is capable of binding multiple antigens that carry the cross-reactive epitopes. Such antigen binding protein binding sites and/or antigen binding proteins with specific binding cross-reactive epitopes are also referred to as antigen binding proteins having multispecific or cross specific binding site, respectively. For example, an antigen binding protein can have a multispecific binding site that specifically binds to epitopes that cross-react with multiple different antigens.

In the present application, the term "subject" typically refers to human or non-human animals, including but not limited to cats, dogs, horses, pigs, cows, sheep, rabbits, mice, rats, or monkeys.

In the present application, the protein, peptide, and/or amino acid sequences involved should also be understood to include at least the following scope: variants or homologs that have the same or similar functions as the protein or peptide.

In the present application, the variant can be, for example, the protein and/or the polypeptide (for example, an antibody or its fragments that specifically binds to pneumolysin protein) that has undergone substitution, deletion, or addition of one or more amino acids in the amino acid sequence of the protein or polypeptide. For example, the functional variant may include a protein or polypeptide that has undergone amino acid changes through at least one, such as 1-30, 1-20, or 1-10, and also one, two, three, four, or five amino acid substitutions, deletions, and/or insertions. The functional variant can essentially maintain the biological characteristics of the protein or peptide prior to modification (such as substitution, deletion, or addition).For example, the functional variant can maintain at least 60%, 70%, 80%, 90%, or 100% of the biological activity (such as antigen binding ability) of the protein or polypeptide before the change. For example, the substitution can be conservative.

In the present application, the homolog can be a protein and/or polypeptide that has at least about 85% (for example, at least about 85%, about 90%, about 91%, about 92%, about 93%, about 94%, about 95%, about 96%, about 97%, about 98%, about 99% or more) sequence homology with the protein and/or polypeptide (e.g., an antibody or fragments thereof that specifically binds to pneumolysin protein).

In the present application, the homology usually refers to the similarity, or association between two or more sequences. The "sequence homology percentage" can be calculated by comparing the two sequences to be compared in the comparison window, determining the number of positions with the same nucleic acid base (such as A, T, C, G, I) or amino acid residue (such as Ala, Pro, Ser, Thr, Gly, Val, Leu, Ile, Phe, Tyr, Trp, Lys, Arg, His, Asp, Glu, Asn, Gln, Cys, and Met) in the two sequences to obtain the number of matching positions, dividing the number of matching positions by the total number of positions in the comparison window (i.e. window size), and multiplying the result by 100, to produce the sequence homology percentage. The comparison to determine the sequence homology percentage can be conducted in many ways known in the art, for example, using a publicly available computer software such as BLAST, BLAST-2, ALIGN or Megalign (DNASTAR) software. Those skilled in the art can determine suitable parameters for sequence alignment, including any algorithm required to achieve maximum alignment within the full-length sequence range being compared or within the target sequence region. The homology can also be determined by the following methods: FASTA and BLAST. For the description of FASTA algorithm, see W.R. Pearson and D.J. Lipman, "Improved Tool for Biological Sequence Comparison", Proc. Satl. Acad. Sci., 85:2444-2448, 1988; and D.J. Lipman and W.R. Pearson, "Rapid and Sensitive Protein Similarity Search", Science, 227:1435-1441,1989.For the description of BLAST algorithm, see S. Altschul, W. Gish, W. Miller, E.W. Myers, and D. Lipman, "A Basic Local Alignment Search Tool", Journal of Molecular Biology, 215:403-410, 1990.

In the present application, the term "comprise" usually refers to the meaning of including, containing, having, or comprising. In some cases, it also means "being" or "consisting of".

In the present application, the term "about" usually refers to changes within a range of 0.5% to 10% above or below the specified value, such as changes within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### Summary of the Invention

### Isolated Antigen Binding Proteins

The CDRs of an antibody, also known as the complementarity determining regions, are part of the variable region. The amino acid residues in this region can contact antigen or epitope. The CDRs of an antibody can be determined through various coding systems, such as CCG, Kabat, Chothia, IMGT, AbM, and combined Kabat/Chothia. These coding systems are known in the art and can be found in, for example, http://www.bioinf.org.uk/abs/index.html#kabatnum.Those skilled in the art can determine the CDR region using different coding systems based on the sequence and structure of the antibody. Based on different coding systems, CDR regions may be different. In the present application, the CDRs covers CDR sequences obtained by any antibody numbering scheme. Variants of the CDRs are also included, which comprises substitution, deletion, and/or addition of one or more residues in the amino acid sequence of a CDR. For example, 1-30, 1-20, or 1-10, and for example, 1, 2, 3, 4, 5, 6, 7, 8, or 9 amino acid substitutions, deletions, and/or insertions can be comprised. Homologues of the CDRs are also included, which comprise amino acid sequences having at least about 85% (for example, have at least about 85%, about 90%, about 91%, about 92%, about 93%, about 94%, about 95%, about 96%, about 97%, about 98%, about 99% or more) sequence homology with the amino acid sequence of the CDRs. In the present application, the isolated antigen binding protein can be defined by IMGT.

In one aspect, the present invention provides an isolated antigen-binding protein comprising at least one CDR in the heavy chain variable region VH of an antibody , the VH comprising the amino acid sequence shown in SEQ ID NO: 39.For example, the VH can comprise the amino acid sequence shown in SEQ ID NO: 22 or SEQ ID NO: 24.In the present application, the HCDRs of the isolated antigen-binding protein can be obtained by any antibody numbering scheme, as long as the VH is the same as the amino acid sequence shown in any one of SEQ ID NO: 39; the HCDRs obtained by any antibody numbering scheme fall within the scope of the present application.

For example, by dividing the amino acid sequences shown in SEQ ID NO: 22 or SEQ ID NO: 24 using the method (scheme) in Table 1, the isolated antigen binding protein in the present application may contain CDRs as shown in the following table.

**Table1. CDR sequences obtained by dividing the amino acid sequences shown in SEQ ID NO: 22 or SEQ ID NO: 24 based on different numbering scheme**

| **Method** | **CDR1** | **CDR2** | **CDR3** |
|---|---|---|---|
| IMGT | GYTFGDNW (SEQ ID NO: 3) | IRLKSNNYAT (SEQ ID NO: 2) | TRTQFITSVVAPF DF (SEQ ID NO: 1) |
| Kabat | DNWMN (SEQ ID NO: 41) | EIRLKSNNYATRYAESV KG (SEQ ID NO: 42) | TQFITS (SEQ ID NO: 43) |
| combina tion | GYTFGDNWMN (SEQ ID NO: 46) | EIRLKSNNYATRYAESV KG (SEQ ID NO: 42) | TRTQFITSVVAPF DF (SEQ ID NO: 1) |

In the present application, the isolated antigen binding protein can contain HCDR3 that comprises an amino acid sequence shown in SEQ ID NO: 1.

In the present application, the isolated antigen binding protein can contain HCDR2 that comprises an amino acid sequence shown in SEQ ID NO: 2.

In the present application, the isolated antigen binding protein contains HCDR1 that comprises an amino acid sequence shown in SEQ ID NO: 3.

In the present application, the isolated antigen binding protein comprises HCDR1, HCDR2 and HCDR3, the HCDR1 comprises an amino acid sequence shown in SEQ ID NO: 3, the HCDR2 comprises an amino acid sequence shown in SEQ ID NO: 2, and the HCDR3 comprises an amino acid sequence shown in SEQ ID NO: 1.

In the present application, the isolated antigen binding protein comprises H-FR1, the C terminus of which is directly or indirectly linked to the N terminus of the HCDR1, and the H-FR1 comprises an amino acid sequence shown in SEQ ID NO: 32.

In the present application, the H-FR1 of the isolated antigen binding protein may comprise the amino acid sequence shown in SEQ ID NO:7 or SEQ ID NO:15.

In the present application, the isolated antigen binding protein comprises H-FR2, the H-FR2 is located between the HCDR1 and HCDR2, and the H-FR2 comprises an amino acid sequence shown in SEQ ID NO: 33.

In the present application, the H-FR2 of the isolated antigen binding protein may comprise the amino acid sequence shown in SEQ ID NO:8 or SEQ ID NO:16.

In the present application, the isolated antigen binding protein comprises H-FR3, the H-FR3 is located between the HCDR2 and HCDR3, and the H-FR3 comprises an amino acid sequence shown in SEQ ID NO: 34.

In the present application, the H-FR3 of the isolated antigen binding protein may comprise the amino acid sequence shown in SEQ ID NO:9 or SEQ ID NO:17.

In the present application, the isolated antigen binding protein comprises H-FR4, the N terminus of which is directly or indirectly linked to the C terminus of the HCDR3, and the H-FR4 comprises an amino acid sequence shown in SEQ ID NO: 35.

In the present application, the H-FR4 of the isolated antigen binding protein may comprise the amino acid sequence shown in SEQ ID NO:10 or SEQ ID NO:18.

In the present application, the isolated antigen binding protein comprises H-FR1, H-FR2, H-FR3 and H-FR4, the H-FR1 comprises an amino acid sequence shown in SEQ ID NO: 32, the H-FR2 comprises an amino acid sequence shown in SEQ ID NO: 33, the H-FR3 comprises an amino acid sequence shown in SEQ ID NO: 34, and the H-FR4 comprises an amino acid sequence shown in SEQ ID NO: 35.For example, the H-FR1 may comprise an amino acid sequence shown in SEQ ID NO: 7 or SEQ ID NO: 15, the H-FR2 may comprise an amino acid sequence shown in SEQ ID NO: 8 or SEQ ID NO: 16, the H-FR3 may comprise an amino acid sequence shown in SEQ ID NO: 9 or SEQ ID NO: 17, and the H-FR4 may comprise an amino acid sequence shown in SEQ ID NO: 10 or SEQ ID NO: 18.For example, the H-FR1 may comprise an amino acid sequence shown in SEQ ID NO: 7, the H-FR2 may comprise an amino acid sequence shown in SEQ ID NO: 8, the H-FR3 may comprise an amino acid sequence shown in SEQ ID NO: 9, and the H-FR4 may comprise an amino acid sequence shown in SEQ ID NO: 10.For example, the H-FR1 may comprise an amino acid sequence shown in SEQ ID NO: 15, the H-FR2 may comprise an amino acid sequence shown in SEQ ID NO: 16, the H-FR3 may comprise an amino acid sequence shown in SEQ ID NO: 17, and the H-FR4 may comprise an amino acid sequence shown in SEQ ID NO: 18.

In the present application, the isolated antigen binding protein may also comprise a HCDR1, HCDR2, HCDR3, H-FR1, H-FR2, H-FR3 and H-FR4.For example, the HCDR1, HCDR2, HCDR3, H-FR1, H-FR2, H-FR3 and H-FR4 of the isolated antigen binding proteins may sequentially comprise the amino acid sequences shown in SEQ ID NO: 3, SEQ ID NO: 2, SEQ ID NO: 1, SEQ ID NO: 7, SEQ ID NO: 8, SEQ ID NO: 9 and SEQ ID NO: 10, respectively. For example, the HCDR1, HCDR2, HCDR3, H-FR1, H-FR2, H-FR3 and H-FR4 of the isolated antigen binding proteins may sequentially comprise the amino acid sequences shown in SEQ ID NO: 3, SEQ ID NO: 2, SEQ ID NO: 1, SEQ ID NO: 15, SEQ ID NO: 16, SEQ ID NO: 17 and SEQ ID NO: 18, respectively.

In the present application, the isolated antigen binding protein contains VH that comprises an amino acid sequence shown in SEQ ID NO: 39.For example, the VH can comprise the amino acid sequence shown in SEQ ID NO: 22.For example, the VH can comprise the amino acid sequence shown in SEQ ID NO: 24.

In the present application, the isolated antigen binding protein may comprise an antibody heavy chain constant region. For example, the heavy chain constant region of an antibody may be derived from the heavy chain constant region of any immunoglobulin, including IgM, IgD, IgG, IgA, and IgE. For example, the heavy chain constant region can derive from human IgG heavy constant region. In the present application, the heavy chain constant region of the immunoglobulin can include its mutant. In the present application, the heavy chain constant region of an antibody may be derived from the heavy chain constant region of any human IgG1-4.For example, the isolated antigen binding protein can be derived from a human IgG1 heavy chain constant region. For example, the heavy chain constant region may comprise an amino acid sequence as shown in SEQ ID NO: 26.

In the present application, the isolated antigen binding protein may comprise heavy chain. For example, the heavy chain may comprise an amino acid sequence as shown in SEQ ID NO: 28. For example, the heavy chain may comprise an amino acid sequence as shown in SEQ ID NO: 30.

In the present application, the isolated antigen binding protein comprises at least one CDR in the light chain variable region VL of an antibody, the VL comprises an amino acid sequence shown in SEQ ID NO: 40.For example, the VL can comprise the amino acid sequence shown in SEQ ID NO: 23 or SEQ ID NO: 25.In the present application, the LCDRs of the isolated antigen-binding protein can be obtained by any antibody numbering scheme, as long as the VL is the same as the amino acid sequence shown in any one of SEQ ID NO: 40; the LCDRs obtained by any antibody numbering scheme fall within the scope of the present application.

For example, by dividing the amino acid sequences shown in SEQ ID NO: 23 or SEQ ID NO: 25 using the method (scheme) in Table 2, the isolated antigen binding protein in the present application may contain CDRs as shown in the following table.

**Table2. CDR sequences obtained by dividing the amino acid sequences shown in SEQ ID NO: 23 or SEQ ID NO: 25 by different numbering scheme**

| **Method** | **CDR1** | **CDR2** | **CDR3** |
|---|---|---|---|
| IMGT | QSLLYSRNQKNY (SEQ ID NO: 6) | WA (SEQ ID NO: 5) | QQYYRYPLT (SEQ ID NO: 4) |
| Kabat | KSSQSLLYSRNQKNY LA (SEQ ID NO: 44) | WASTRES (SEQ ID NO: 45) | QQYYRYPLT (SEQ ID NO: 4) |
| combina tion | KSSQSLLYSRNQKNY LA (SEQ ID NO: 44) | WASTRES (SEQ ID NO: 45) | QQYYRYPLT (SEQ ID NO: 4) |

In the present application, the isolated antigen binding protein contains LCDR3 that comprises an amino acid sequence shown in SEQ ID NO: 4.

In the present application, the isolated antigen binding protein contains LCDR2 that comprises an amino acid sequence shown in SEQ ID NO: 5.

In the present application, the isolated antigen binding protein contains LCDR1 that comprises an amino acid sequence shown in SEQ ID NO: 6.

In the present application, the isolated antigen binding protein comprises LCDR1, LCDR2 and LCDR3, the LCDR1 comprises an amino acid sequence shown in SEQ ID NO: 6, the LCDR2 comprises an amino acid sequence shown in SEQ ID NO: 5, and the LCDR3 comprises an amino acid sequence shown in SEQ ID NO: 4.

In the present application, the isolated antigen binding protein comprises L-FR1, the C terminus of which is directly or indirectly linked to the N terminus of the LCDR1, and the L-FR1 comprises an amino acid sequence shown in SEQ ID NO: 36.For example, the L-FR1 may comprise the amino acid sequence shown in SEQ ID NO:11.For example, the L-FR1 may comprise the amino acid sequence shown in SEQ ID NO:19.

In the present application, the isolated antigen binding protein comprises L-FR2, the L-FR2 is located between the LCDR1 and LCDR2, and the L-FR2 comprises an amino acid sequence shown in SEQ ID NO: 37.For example, the L-FR2 may comprise the amino acid sequence shown in SEQ ID NO:12.For example, the L-FR2 may comprise the amino acid sequence shown in SEQ ID NO:20.

In the present application, the isolated antigen binding protein comprises L-FR3, the L-FR3 is located between the LCDR2 and LCDR3, and the L-FR3 comprises an amino acid sequence shown in SEQ ID NO: 38.For example, the L-FR3 may comprise the amino acid sequence shown in SEQ ID NO:13.For example, the L-FR3 may comprise the amino acid sequence shown in SEQ ID NO:21.

In the present application, the isolated antigen binding protein comprises L-FR4, the N terminus of which is directly or indirectly linked to the C terminus of the LCDR3, and the L-FR4 comprises an amino acid sequence shown in SEQ ID NO: 14.

In the present application, the isolated antigen binding protein comprises L-FR1, L-FR2, L-FR3 and L-FR4, the L-FR1 comprises an amino acid sequence shown in SEQ ID NO: 36, the L-FR2 comprises an amino acid sequence shown in SEQ ID NO: 37, the L-FR3 comprises an amino acid sequence shown in SEQ ID NO: 38, and the L-FR4 comprises an amino acid sequence shown in SEQ ID NO: 14.For example, the L-FR1 may comprise an amino acid sequence shown in SEQ ID NO: 11 or SEQ ID NO: 19, the L-FR2 may comprise an amino acid sequence shown in SEQ ID NO: 12 or SEQ ID NO: 20, the L-FR3 may comprise an amino acid sequence shown in SEQ ID NO: 13 or SEQ ID NO: 21, and the L-FR4 may comprise an amino acid sequence shown in SEQ ID NO: 14.For example, the L-FR1 may comprise an amino acid sequence shown in SEQ ID NO: 11, the L-FR2 may comprise an amino acid sequence shown in SEQ ID NO: 12, the L-FR3 may comprise an amino acid sequence shown in SEQ ID NO: 13, and the L-FR4 may comprise an amino acid sequence shown in SEQ ID NO: 14.For example, the L-FR1 may comprise an amino acid sequence shown in SEQ ID NO: 19, the L-FR2 may comprise an amino acid sequence shown in SEQ ID NO: 20, the L-FR3 may comprise an amino acid sequence shown in SEQ ID NO: 21, and the L-FR4 may comprise an amino acid sequence shown in SEQ ID NO: 14.

In the present application, the isolated antigen binding protein may also comprise a LCDR1, LCDR2, LCDR3, L-FR1, L-FR2, L-FR3 and L-FR4.For example, the LCDR1, LCDR2, LCDR3, L-FR1, L-FR2, L-FR3 and L-FR4 of the isolated antigen binding proteins may comprise the amino acid sequences shown in SEQ ID NO: 6, SEQ ID NO: 5, SEQ ID NO: 4, SEQ ID NO: 11, SEQ ID NO: 12, SEQ ID NO: 13 and SEQ ID NO: 14, respectively. For example, the LCDR1, LCDR2, LCDR3, L-FR1, L-FR2, L-FR3 and L-FR4 of the isolated antigen binding proteins may comprise the amino acid sequences shown in SEQ ID NO: 6, SEQ ID NO: 5, SEQ ID NO: 4, SEQ ID NO: 19, SEQ ID NO: 20, SEQ ID NO: 21 and SEQ ID NO: 14, respectively.

In the present application, the isolated antigen binding protein contains VL that comprises an amino acid sequence shown in SEQ ID NO: 40.For example, the light chain may comprise an amino acid sequence as shown in SEQ ID NO: 23.For example, the light chain may comprise an amino acid sequence as shown in SEQ ID NO: 25.

In the present application, the isolated antigen binding protein may comprise an antibody light chain constant region. For example, the light chain constant region can derive from human antibody light chain constant region. For example, the light chain constant region can derive from human Igκ constant region. For example, the light chain constant region may comprise an amino acid sequence as shown in SEQ ID NO: 27.

In the present application, the isolated antigen binding protein may comprise light chain. For example, the light chain of the isolated antigen binding protein may comprise an amino acid sequence as shown in SEQ ID NO: 29.For example, the light chain of the isolated antigen binding protein may comprise an amino acid sequence as shown in SEQ ID NO: 31.

In the present application, the isolated antigen binding protein may comprise HCDR1, HCDR2, HCDR3, LCDR1, LCDR2, and LCDR3.For example, the HCDR1 of the isolated antigen binding protein comprises an amino acid sequence shown in SEQ ID NO: 3, the HCDR2 comprises an amino acid sequence shown in SEQ ID NO: 2, and the HCDR3 comprises an amino acid sequence shown in SEQ ID NO: 1, the LCDR1 comprises an amino acid sequence shown in SEQ ID NO: 6, the LCDR2 comprises an amino acid sequence shown in SEQ ID NO: 5, and the LCDR3 comprises an amino acid sequence shown in SEQ ID NO: 4.

In the present application, the isolated antigen binding protein may comprise VH and VL. For example, the VH comprises an amino acid sequence shown in SEQ ID NO: 22, and the VL comprises an amino acid sequence shown in SEQ ID NO: 23.For example, the VH comprises an amino acid sequence shown in SEQ ID NO: 24, and the VL comprises an amino acid sequence shown in SEQ ID NO: 25.

In the present application, the isolated antigen binding protein may comprise antibodies or their antigen binding fragments. In the present application, the antigen binding fragment may include Fab, Fab', Fv fragment, F(ab')2, F(ab)2, scFv, di-scFv and/or dAb. In the present application, the antibodies may include monoclonal antibodies, chimeric antibodies, humanized antibodies, and/or fully human antibodies. For example, the VH of the humanized antigen binding protein may comprise an amino acid sequence shown in SEQ ID NO: 24.For example, the VL of the humanized antigen binding protein may comprise an amino acid sequence shown in SEQ ID NO: 25.

In addition, it should be noted that the isolated antigen binding protein described herein may comprise heavy chain and/or light chain sequences with one or more conserved sequence modifications. The so-called "conserved sequence modification" refers to an amino acid modification that will not significantly affect or change the binding characteristics of antibodies. Such conserved modifications include substitution, addition, and deletion of amino acids. The modification may be introduced into the isolated antigen binding protein described herein through standard technologies known in the art, such as point mutation and PCR mediated mutation. Conserved amino acid substitution refers to the replacement of amino acid residues with amino acid residues with similar side chains. Amino acid residues with similar side chains are known in the field. These amino acid residue groups include those with a basic side chain (e.g., lysine, arginine, histidine), an acidic side chain (e.g., aspartic acid, glutamic acid), a uncharged polar side chain (e.g., glycine, asparagine, glutamine, serine, threonine, tyrosine, cysteine, tryptophan), a non-polar side chains (e.g., alanine, valine, leucine, isoleucine, proline, phenylalanine, methionine), a β-branched side chain (e.g., threonine, valine, isoleucine) and an aromatic side chain (e.g., tyrosine, phenylalanine, tryptophan, histidine).In certain embodiments, one or more amino acid residues in the CDR region of the isolated antigen binding protein described herein can be replaced with another amino acid residues in the same side chain group. Those skilled in the art know that some conserved modification of sequence will not destroy antigen binding. For details, see, for example, Brummell et. al., (1993) Biochem 32:1180-8; de Wildt et. al., (1997) Prot. Eng. 10:835-41; Komisarov et. al., (1997) J. Biol. Chem. 272:26864-26870; Hall et. al., (1992) J. Immunol. 149:1605-12; Kelley and O'Connell (1993) Biochem. 32:6862-35; Adib Conquy et. al., (1998) Int. Immunol. 10:341-6 and Beers et. al., (2000) Clin. Can. Res. 6:2835-43.

In the present application, antigen binding proteins with affinity for pneumolysin protein antigen can be screened by immunizing mice with pneumolysin mutants, and fusing mouse splenocytes and myeloma cells. In the present application, the post-translational modification site of the screened antigen-binding protein can also be optimized to obtain a humanized sequence.

The antigen binding protein against pneumolysin described herein may be identified, screened, or characterized by various known assays in the art.

For example, known methods such as ELISA (ELISA), immunoblotting (e.g., Western blotting), flow cytometry (e.g., FACS), immunohistochemistry, immunofluorescence, etc. may be used to test the antigen binding activity of the antigen binding protein.

In the present application, the isolated antigen binding protein may specifically bind to pneumolysin. In the present application, the pneumolysin comprises wild-type pneumolysin and variants thereof. In the present application, the variants of the pneumolysin comprises wild-type pneumolysin which amino acid residue 146 is deleted. In the present application, the amino acid sequence of wild-type pneumolysin can be determined from the Uniprot database (Entry ID: Q04IN8).

In the present application, the binding of the isolated antigen-binding protein to pneumolysin can be detected by ELISA. For example, the antigen-binding proteins described herein may bind to pneumolysin at a EC₅₀ less than or equal to about 0.03 µg/ml, less than or equal to about 0.028 µg/ml, less than or equal to about 0.026 µg/ml, less than or equal to about 0.024 µg/ml, less than or equal to about 0.022 µg/ml, less than or equal to about 0.02034 µg/ml, less than or equal to about 0.020 µg/ml, less than or equal to about 0.018 µg/ml, less than or equal to about 0.01706 µg/ml, less than or equal to about 0.016 µg/ml, less than or equal to about 0.014 µg/ml, less than or equal to about 0.012 µg/ml, less than or equal to about 0.010 µg/ml.

In the present application, the isolated antigen binding protein can neutralize the biological activity of the pneumolysin protein. For example, the antihemolytic activity of the antigen binding protein can be determined by mixing the recombinant pneumolysin with the isolated antigen binding protein described herein, adding rabbit red blood cells, and detecting the antihemolytic activity.

In the present application, the isolated antigen binding protein can prevent and/or treat diseases and/or conditions .In the present application, the diseases and/or conditions may be caused or mediated by Streptococcus pneumonia the present application, the diseases and/or conditions comprise complications of the diseases and/or conditions caused or mediated by *Streptococcus pneumoniae.* For example, the disease and/or condition may comprise sepsis and/or bacteremia.

Polypeptide molecules, nucleic acid molecules, vectors, cells, immunoconjugates and pharmaceutical compositions

In another aspect, the present application provides polypeptide molecules that may comprise the isolated antigen binding proteins described herein.

In the present application, the polypeptide molecule may comprise a fusion protein. In the present application, the polypeptide molecule may be a fusion protein. In the present application, the fusion protein may comprise a multispecific antibody.

In one aspect, the present application provides an isolated nucleic acid molecule that may encode the isolated antigen binding protein described herein. For example, it can be produced or synthesized by the following methods: (i) *in vitro* amplification, such as polymerase chain reaction (PCR) amplification; (ii) cloning and recombination; (iii) purification, for example, by enzyme digestion and gel electrophoresis fractionation; or (iv) synthesis, such as by chemical synthesis.

In another aspect, the present application provides a vector that may comprise the nucleic acid molecules described herein. In addition, the vector may also comprise other genes, such as labelling genes that allow selection of the vector in appropriate host cells and under appropriate conditions. In addition, the vector may also include an expression control element that allows the coding region to be correctly expressed in an appropriate host. Such control element is familiar to those skilled in the art, for example, it may comprise promoter, ribosome-binding site, enhancer, and other control elements that regulate gene transcription or mRNA translation. The vector can transform, transduce or transfect a host cell, so that the genetic material elements carried by the vector can be expressed in the host cell. The vectors may include, for example, plasmids, cosmids, viruses, bacteriophages, or other vectors commonly used in genetic engineering. For example, the vector is an expression vector. In addition, the vector may also include components that assist it in entering cells, such as viral particles, liposomes, or protein coats, but not limited to these substances.

In another aspect, the present application provides a cell that may comprise the nucleic acid molecule, or the vector described herein. In certain embodiments, each or every host cell may comprise one or more nucleic acid molecules or vectors described herein. In certain embodiments, each or every host cell may comprise multiple number of (e.g., two or more number of) or multiple (e.g., two or more) nucleic acid molecules or vectors described herein. For example, the vector described herein may be introduced into host cells, such as eukaryotic cells, such as cells from plants, fungi, or yeast cells. In some embodiments, the cells may be bacterial cells (e.g., Escherichia coli), yeast cells or other eukaryotic cells, such as COS cells, Chinese hamster ovary (CHO) cells, CHO-K1 cells, LNCAP cells, HeLa cells, 293T cells, COS-1 cells, SP2/0 cells, NS0 cells or myeloma cells. The vector described herein may be introduced into the host cells through methods known in the art, such as electroporation, lipofectine transfection, lipofectamine transfection, etc.

In another aspect, the present application also provides an immunoconjugate, which may comprise the isolated antigen binding protein described herein.

In some embodiments, the isolated antigen binding proteins or their fragments described herein may be linked to another reagent, such as a chemotherapeutic agent, toxin, immunotherapeutic agent, imaging probe, spectroscopic probe, etc. The linkage may be through one or more covalent bond, or non-covalent interactions, and may include chelation. Several linkers may be used (which are known in the art) to form immunoconjugates. In addition, immunoconjugates can be provided in the form of fusion proteins, which can be expressed by polynucleotides encoding immunoconjugates. The immunoconjugate may also include, for example, an antibody drug conjugate (ADC). In ADC, antibodies and therapeutic agents can be cross-linked through linkers that can be cleaved, such as peptide linkers, disulfide linkers, or hydrazone linkers.

In another aspect, the present application also provides a pharmaceutical composition, which may include the isolated antigen binding protein, polypeptide molecule, immunoconjugate, nucleic acid molecule, vector and/or cell described herein, as well as optionally a pharmaceutically acceptable vehicle.

In certain embodiments, the pharmaceutical composition may also include one or more (pharmaceutically effective) suitable reagents of adjuvants, stabilizers, excipients, diluents, solubilizers, surfactants, emulsifiers, and/or preservatives. The acceptable components of the composition are preferably non-toxic to the recipient at the dosage and concentration used. The pharmaceutical compositions of the present invention include but are not limited to liquid, frozen, and lyophilized compositions.

In certain embodiments, the pharmaceutical composition may also comprise more than one active compound, typically those with complementary activity that do not adversely affect each other. The type and effective amount of such drugs may depend on, for example, the amount and type of antagonists present in the formulation, as well as the clinical parameters of the subjects.

In certain embodiments, the pharmaceutically acceptable vehicle may include any and all solvents, dispersants, coatings, isotopes, and absorption delay agents compatible with drug administration, typically safe and non-toxic.

In certain embodiments, the pharmaceutical composition may include parenteral, percutaneous, intracavitary, intra-arterial, intrathecal, and/or intranasal administration or direct injection into tissues. For example, the pharmaceutical composition can be administered to patients or subjects through infusion or injection. In certain embodiments, the administration of the pharmaceutical composition can be carried out in different ways, such as intravenous, intraperitoneal, subcutaneous, intramuscular, local, or dermal administration. In certain embodiments, the pharmaceutical composition may be administered continuously. The uninterrupted (or continuous) administration may be achieved through a small pump system worn by the patient to measure the therapeutic agent flowing into the patient's body, as described in WO2015/036583.

### Methods of preparation

In another aspect, the present application provides a method for preparing the isolated antigen binding protein. The method may comprise culturing the host cell described herein under the conditions for expressing the antigen binding protein. For example, by using appropriate culture media, appropriate temperature, and culture time, these methods may be understood by those skilled in the art.

Any method suitable for producing monoclonal antibodies can be used to produce the antigen binding protein of the present application. For example, animals can be immunized with an attenuated strain of pneumolysin or a fragment thereof. For example, the attenuated strain of *Streptococcus pneumonia* may comprise a deletion mutation at position 146 of pneumolysin compared to the wild type. Suitable immunization methods may be used, including adjuvants, immunostimulants, repeated booster immunization, and one or more pathways may be used.

Any suitable form of pneumolysin may be used as immunogens (antigens) to produce non-human antibodies specific for pneumolysin, and to screen the biological activity of the antibodies. For example, the trigger immunogen can be full-length pneumolysin, including natural homodimers or peptides containing single/multiple epitopes. Immunogens can be used alone or in combination with one or more known immunogenic enhancers in the art.

### Method and Use

In another aspect, the present application provides use of the isolated antigen binding protein, polypeptide molecule, nucleic acid molecule, vector, cell, immunoconjugate, and/or pharmaceutical combination in the manufacture of a medicament for prevention and/or treatment of diseases and/or conditions.

In another aspect, the present application also provides a method for preventing and/or treating diseases and/or conditions, which may comprise administering the isolated antigen binding protein, polypeptide molecule, nucleic acid molecule, vector, cell, immunoconjugate, and/or pharmaceutical composition described herein to a subject in need.

In the present application, the administration may be carried out in different ways, such as intravenous, intratumoral, intraperitoneal, subcutaneous, intramuscular, local or intradermal administration.

In another aspect, the isolated antigen binding protein, polypeptide molecule, nucleic acid molecule, vector, cell, immunoconjugate, and/or pharmaceutical composition described herein may be used for preventing and/or treating diseases and/or conditions.

In the present application, the diseases and/or conditions may be caused or mediated by *Streptococcus pneumoniae.*

In the present application, the diseases and/or conditions comprise complications of the diseases and/or conditions caused or mediated by *Streptococcus pneumoniae.*

In the present application, the diseases and/or conditions include bacterial infections.

In the present application, the diseases and/or conditions include bacteremia and/or sepsis.

In the present application, the isolated antigen binding protein, polypeptide molecule, nucleic acid molecule, vector, cell, immunoconjugate, and/or pharmaceutical composition described herein can be used alone or in combination with other drugs to prevent and/or treat diseases and/or conditions. In certain embodiments, the other drugs may be any currently known drug with antibacterial effects. The diseases and/or conditions may be caused by *Streptococcus pneumoniae* infection or related diseases caused by other infections.

In another aspect, the present application also provides a method for detecting pneumolysin protein in a sample, wherein the method comprises using the isolated antigen binding protein, the polypeptide molecule, the nucleic acid molecule, the vector, the cell, the immunoconjugate, and/or the pharmaceutical composition.

In the present application, the method for detecting pneumolysin protein in a sample may be an *in vitro* method. For example, the presence and/or content of pneumolysin in the sample can be detected by contacting the isolated antigen binding protein described herein with an ex vivo sample. In some cases, the method for detecting pneumolysin in a sample is not for therapeutic purposes. In some cases, the method for detecting pneumolysin in a sample is not a diagnostic method.

In another aspect, the present application also provides a reagent or kit for detecting pneumolysin protein in a sample comprising the isolated antigen binding protein, polypeptide molecule, nucleic acid molecule, vector, cell, immunoconjugate, and/or pharmaceutical composition.

In another aspect, the present application also provides use of the isolated antigen binding protein, the polypeptide molecule, the nucleic acid molecule, the vector, the cell, the immunoconjugate and/or the pharmaceutical composition in the manufacture of a kit for detecting the presence and/or content of pneumolysin in a sample.

Without being limited by any theory, the following examples are only intended to illustrate the antigen binding protein, preparation method, and use of the present application, and are not intended to limit the scope of the present invention.

### Example

### Example 1, Expression of pneumolysin (PLY) recombinant protein

The amino acid sequence of pneumolysin was obtained from the Uniprot database (PLY, Entry ID: Q04IN8), and the alanine at position 146 according to the full-length amino acid sequence of pneumolysin was deleted to obtain the amino acid of the attenuated PLY mut. The amino acid sequence was used as the template; the restriction enzyme cleavage site was NdeI/XhoI; the His tag was introduced at C-terminus; codon optimization and gene synthesis was finished by Beijing Qingke Biotechnology Co., Ltd. to obtain the plasmids of PLY and PLY mut proteins. The recombinant plasmid was transformed into E. coli BL21 (DE3) plysS, and the E. coli expression system was used to recombinantly express PLY and PLY mut recombinant proteins.

### Example 2, Immunization of Balb/C mice with PLY mut antigen

Five Balb/C mice were immunized by subcutaneous multi-point immunization of the prepared PLY mut protein. The subcutaneous multi-point immunization process is shown below.

| Immunization scheme | |
|---|---|
| First immunizatio n | 50ul of blood was collected before immunization as negative serum; The adjuvant was mixed with PLYmut antigen (1mg) at a ratio of 1:1, emulsified, and then injected at multiple points internally and subcutaneously |
| Second immunizatio n | The adjuvant was mixed with PLYmut antigen (0.5mg) at a ratio of 1:1, emulsified, and then injected at multiple points internally and subcutaneously |
| Third immunizatio n | The adjuvant was mixed with PLYmut antigen (0.5mg) at a ratio of 1:1, emulsified, and then injected at multiple points internally and subcutaneously |
| Titer detection | One week after the third immunization, the serum of the immunized mice was taken for titer detection |
| Booster immunizatio n | One week after titer detection, PLYmut antigen (2mg) was used and injected into tail vein |
| | Three days after the booster immunization, the mouse spleen was taken for cell fusion |

### Example 3, Fusion of spleen cells of immunized mice with myeloma cells and screening of antigen-binding proteins with PLYmut antigen affinity

By PEG method, Myeloma cells P3X63Ag8.653 were fused with spleen cells of mice immunized with PLY mut antigen. The fused hybridoma cells were seeded in 96-well plates for culture. Hybridoma cells were then cultured using HAT medium and HT medium. When the cell supernatant of the 96-well plate was slightly yellow, 100 µl of the cell culture supernatant was taken to detect the hybridoma clone cells binding the PLY mut antigen by ELISA screened method. After selecting the hybridoma parent clone cells with strong affinity and continuing to culture, subcloning was performed using limited dilution method, and monoclonal cells were selected. Using monoclonal supernatant for screening, 2E5 monoclonal cells secreting antibodies with strong affinity for PLYmut protein were obtained through ELISA method. The secreted monoclonal antibody was named m2E5.

### Example 4, Identification of the sequence of a specific murine antigen-binding protein

The total RNA of 1×10⁶ 2E5 monoclonal cells secreting anti-PLY mut antibody was extracted by RNAfast200 kit (Shanghai Feijie Biotechnology Co., Ltd.).The RNA was reverse transcribed into cDNA using a reverse transcription kit (Takara). cDNA fragments were amplified by primers (Anke Krebber. 1997), PCR products were purified by DNA product purification kit (Beijing Tiangen Biochemical Technology Co., Ltd.), and connected to T vector by ligation kit (Beijing Qingke Biotechnology Co., Ltd.), and introduced into competent cells by transformation. Sequencing was completed by Shanghai Qingke Biotechnology. The sequencing results of the heavy chain variable region (m2E5VH) and light chain variable region (m2E5VL) of the mouse monoclonal antibody m2E5 are as follows.
m2E5 heavy chain variable region (m2E5VH) SEQ ID NO: 22 
EVKVEESGGGSVQPGGSMKLSCVASGYTFGDNWMNWVRQSPEKGLEWVA
EIRLKSNNYATRYAESVKGRFTISRDDSKSSVYLQMNNLRTEDTGIYYCTRTQFITS
VVAPFDFWGQGTTLTVSS
m2E5 light chain variable region (m2E5VL) SEQ ID NO: 23

DIVMSQSPSSLGVSVGEKITMSCKSSQSLLYSRNQKNYLAWYQQKPGQSPKL
LIYWASTRESGVPDRFTGSGSGTDFTLTISSVKAEDLAVYYCQQYYRYPLTFGGGT
KVEIK

Wherein, the underlined part represents the CDR regions generated by the IMGT numbering scheme.

### Example 5, Humanization of anti-PLY murine antigen-binding protein

Through the NCBI database alignment (https://www.ncbi.nlm.nih.gov/igblast/), the germline sequence closest to the mouse antibody 2E5 (m2E5) was selected as the template for the humanization of m2E5. The post-translational modification sites of the antigen-binding protein were optimized, and the sequences of the heavy chain variable region (hu2E5VH3) and light chain variable region (hu2E5VL0) of the obtained humanized antibody 2ESH3L0 were as follows.

Heavy chain variable region (hu2E5VH3) of the humanized antigen binding protein 2E5, SEQ ID NO:24

EVQLVESGGGLVQPGGSLKLSCAASGYTFGDNWMNWVRQASGKGLEWVG
EIRLKSNNYATRYAESVKGRFTISRDDSKNTVYLQMNSLKTEDTAVYYCTRTQFIT
SVVAPFDFWGQGTTVTVSS

Light chain variable region (hu2E5VL0) of humanized antigen binding protein 2E5, SEQ ID NO:25

DIVMTQSPDSLAVSLGERATINCKSSQSLLYSRNQKNYLAWYQQKPGQPPKL
LIYWASTRESGVPDRFSGSGSGTDFTLTISSLQAEDVAVYYCQQYYRPLTFGGGT
KVEIK

Wherein, the underlined part represents the CDR regions generated using the IMGT numbering scheme.

### Example 6, Expression of anti-PLY antigen-binding protein and detection of antigen-binding activity

The C-terminus of the m2E5 heavy chain variable region was linked to the N-terminus of the constant region of human immunoglobulin γ1 (IgG1) and the product was introduced into the pcDNA3.4 vector to obtain the expression vector pcDNA3.4-c2E5VHCH of the heavy chain of 2E5 mouse/human chimeric antibody (2E5HL). The C-terminus of the variable region of the m2E5 light chain was linked to the N-terminus of the kappa constant region of the light chain and the product was introduced into the pcDNA3.4 vector to obtain the expression vector pcDNA3.4-c2E5VLCL of the 2E5 chimeric antibody light chain.

Wherein the amino acid sequence of the heavy chain (c2E5VHCH) and the light chain (c2E5VLCL) of the chimeric antibody 2E5HL are:
c2E5VHCH SEQ ID NO: 28: 
EVKVEESGGGSVQPGGSMKLSCVASGYTFGDNWMNWVRQSPEKGLEWVA
EIRLKSNNYATRYAESVKGRFTISRDDSKSSVYLQMNNLRTEDTGIYYCTRTQFITS
VVAPFDFWGQGTTLTVSSASTKGPSVFPLAPSSKSTSGGTAALGCLVKDYFPEPVT
 
VSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLYITREPEVTCVVVDVSH
EDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEAL
HNHYTQKSLSLSPGK
c2E5VLCL SEQ ID NO: 29:

DIVMSQSPSSLGVSVGEKITMSCKSSQSLLYSRNQKNYLAWYQQKPGQSPKL
LIYWASTRESGVPDRFTGSGSGTDFTLTISSVKAEDLAVYYCQQYYRYPLTFGGGT
KVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGE
C

The C-terminus of the humanized 2E5 heavy chain variable region was linked to the N-terminus of the constant region of human immunoglobulin γ1 (IgG1) and the product was introduced into the pcDNA3.4 vector to obtain the expression vector pcDNA3.4-hu2E5VH3CH of the humanized heavy chain. The C-terminus of the variable region of the 2E5 humanized light chain was linked to the N-terminus of the kappa constant region of the light chain and the product was introduced into the pcDNA3.4 vector to obtain the expression vector pcDNA3.4-hu2E5VL0CL of the humanized light chain.

The amino acid sequence of the heavy chain (hu2E5VH3CH) and the light chain (hu2E5VL0CL) of the humanized antibody 2ESH3L0 are:
hu2E5VH3CH SEQ ID NO: 30: 
EVQLVESGGGLVQPGGSLKLSCAASGYTFGDNWMNWVRQASGKGLEWVG
EIRLKSNNYATRYAESVKGRFTISRDDSKNTVYLQMNSLKTEDTAVYYCTRTQFIT
SVVAPFDFWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTK
VDKRVEPKSCDKTHTCPPCPAPELLGGPSVFLFPPKPKDTLYITREPEVTCVVVDVS
HEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHEA
LHNHYTQKSLSLSPGK
hu2E5VL0CL SEQ ID NO: 31:

DIVMTQSPDSLAVSLGERATINCKSSQSLLYSRNQKNYLAWYQQKPGQPPKL
LIYWASTRESGVPDRFSGSGSGTDFTLTISSLQAEDVAVYYCQQYYRYPLTFGGGT
KVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGE
C

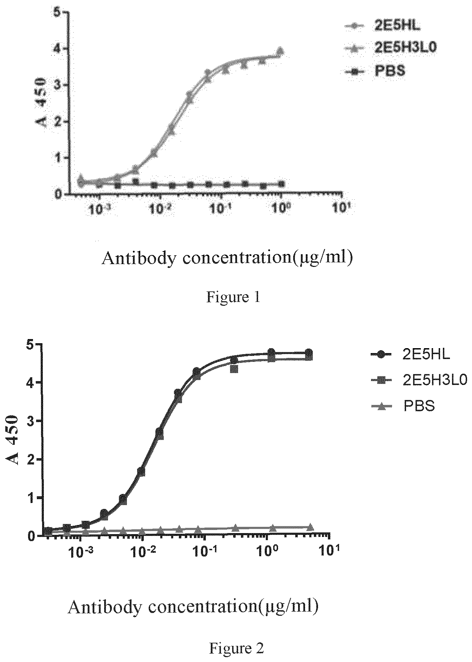

The expression vector pcDNA3.4-m2E5VHCH of the 2E5HL chimeric antibody heavy chain and the expression vector pcDNA3.4-m2E5VLCL of the light chain were co-transfected into Expi293F cells, and 2E5HL was transiently expressed by the Expi293F expression system. Finally, the chimeric antibody protein was purified with Protein A. The expression vector pcDNA3.4-hu2E5VH3CH of the 2E5H3L0 humanized antibody heavy chain and the expression vector pcDNA3.4-hu2E5VL0CL of the light chain were co-transfected into Expi293F cells, and 2E5H3E0 was transiently expressed by the Expi293F expression system. Finally, the humanized antibody protein was purified with Protein A. The purified 2E5HL chimeric antibody and 2E5H3L0 humanized antibody were identified by ELISA for antigen-binding activity: 0.1 µg/well of PLY and PLYmut proteins were coated on a 96-well microtiter plate (Thermo), incubated at 4°C overnight, and washed with PBST buffer. After washing, 200 µl of 5% BSA was added to each well and the plate was incubated at 37°C for 1 hour; after blocking, the plate was washed with PBST buffer; 0.1 µg of 2E5HL human-mouse chimeric antibody or 2ESH3L0 humanized antibody were added to the ELISA plate step by step according to 2 times dilution gradient, incubated at 4°C overnight; The plate was washed after incubating with the primary antibody overnight, and added with 1:10000 diluted HRP-labeled goat anti-human IgG secondary antibody at 100 µl per well, incubated at 37°C for 1 hour and washed; the plate was added with 100 µl TMB per well and incubated at 37°C for color development, which is stopped by adding stop solution after 10 min. The results were read by a microplate reader (Multiskcin FC, Thermo) and plotted by Graphpad software The results of antibody binding to PLY protein are shown in Figure 1. The EC₅₀ of 2E5HL is 0.01706 µg/ml, and the EC₅₀ of 2ESH3L0 is 0.02034 µg/ml; the results of antibody binding to PLYmut protein are shown in Figure 2, and the EC₅₀ of 2E5HL is 0.01582µg/ml, the EC₅₀ of 2ESH3L0 is 0.01617µg/ml. The results showed that both the humanized antibody 2ESH3L0 and the chimeric antibody 2E5HL had strong affinity with pneumolysin PLY and PLYmut proteins.

### Example 7, In vitro neutralization of the activity of pneumolysin (PLY)

6.25ng of recombinant pneumolysin protein and chimeric antibody 2E5HL or humanized antibody 2ESH3L0 were mixed in a certain proportion and added to a 96-well plate (Thermo), incubated at 37°C for 10min, added with 5% rabbit erythrocytes, and incubated at 37°C for 1h.The samples were centrifuged at 3000 rpm for 5 min, and the anti-hemolytic activity was determined by detecting the absorbance at a wavelength of 405 nm using a microplate reader (Multiskcin FC, Thermo).

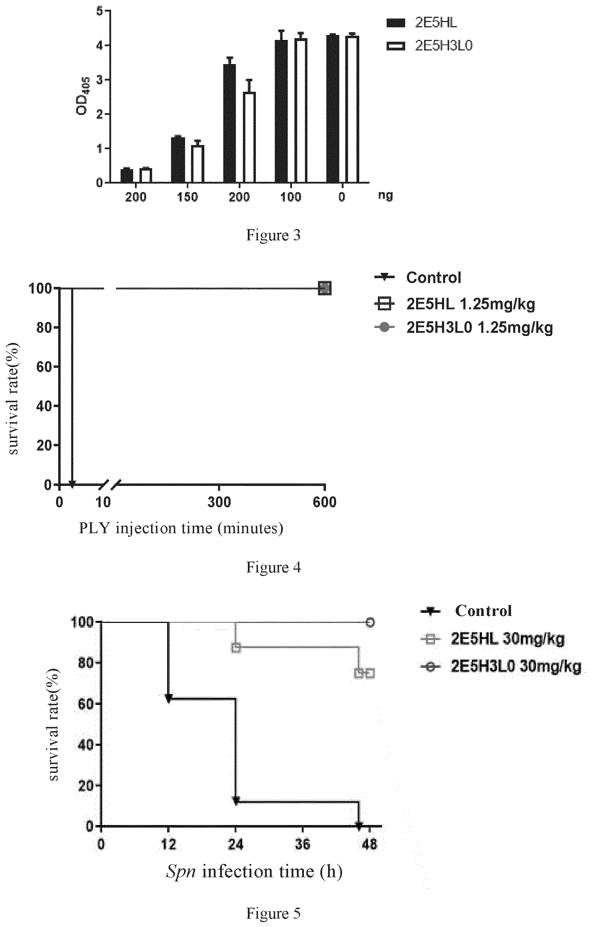

The results are shown in Figure 3. pneumolysin has rabbit erythrocyte hemolytic activity, while both chimeric antibody 2E5HL and humanized antibody 2ESH3L0 can effectively neutralize the rabbit erythrocyte hemolytic activity of pneumolysin in a dose-dependent manner.

### Example 8, the protective effect of the antigen-binding protein described in the present application on PLY toxin model mice.

C57BL/6 mice were pre-injected with 1.25 mg/kg chimeric antibody 2E5HL, humanized antibody 2ESH3L0 and human IgG antibody (as a control), and 1 h later, 0.75 µg pneumolysin PLY protein was injected into the tail vein. The survival time of mice was observed.

The results are shown in Figure 4. All the mice in the control group died, while the mice injected with the chimeric antibody 2E5HL and the humanized antibody 2ESH3L0 were fully protected.

### Example 9, the protective effect of the antigen-binding protein described in the present application on mice infected with Streptococcus pneumoniae.

*Streptococcus pneumoniae* was taken from the bacterial bank and thawed, then 200 µl of the bacterial solution was spread evenly on the Columbia blood plate with a spreader, cultivated for 10 to 12 hours. A piece of blood agar (3cm×3cm) was taken out from the Columbia blood plate, and added to 15 ml sterilized THY medium, incubated at 37°C until the OD₆₀₀ reached 0.4 to 0.6, which took about 2 to 3 hours. Bacteria were collected by centrifugation and resuspended in PBS for use. C57BL/6J mice were randomly divided into model group and monoclonal antibody treatment group according to body weight. Mice were injected with 2×10⁸CFU of *Streptococcus pneumoniae* at the tail vein 2 hours in advance, and 2 hours later, 30 mg/kg of 2E5HL, 2ESH3L0 or human IgG (control) were injected into the tail vein respectively, and the survival of mice in each group was observed and recorded.

The results are shown in Figure 5. Compared with the control human IgG, the chimeric antibody 2E5HL and the humanized antibody 2ESH3L0 have a good protective effect on *Streptococcus pneumoniae* (*Spn*)-infected mice.

## Claims

1. An isolated antigen-binding protein comprising at least one CDR of the heavy chain variable region VH of an antibody , the VH comprising the amino acid sequence shown in SEQ ID NO: 39.

2. The isolated antigen binding protein according to claim 1, which comprises a HCDR3, the HCDR3 comprises an amino acid sequence shown in SEQ ID NO: 1.

3. The isolated antigen binding protein according to any one of claims 1-2, which comprises a HCDR2, the HCDR2 comprises an amino acid sequence shown in SEQ ID NO: 2.

4. The isolated antigen binding protein according to any one of claims 1-3, which comprises a HCDR1, the HCDR1 comprises an amino acid sequence shown in SEQ ID NO: 3.

5. The isolated antigen binding protein according to any one of claims 1-4, comprising HCDR1, HCDR2, and HCDR3, the HCDR1 comprises an amino acid sequence shown in SEQ ID NO: 3, the HCDR2 comprises an amino acid sequence shown in SEQ ID NO: 2, and the HCDR3 comprises an amino acid sequence shown in SEQ ID NO: 1.

6. The isolated antigen binding protein according to any one of claims 4-5, comprising a H-FR1, the C-terminus of the H-FR1 is directly or indirectly linked to the N-terminus of the HCDR1, and the H-FR1 comprises an amino acid sequence shown in SEQ ID NO: 32.

7. The isolated antigen binding protein according to claim 6, wherein the H-FR1 comprises an amino acid sequence shown in SEQ ID NO: 7 or SEQ ID NO: 15.

8. The isolated antigen binding protein according to any one of claims 4-7, which comprises a H-FR2 located between HCDR1 and HCDR2, and the H-FR2 comprises an amino acid sequence shown in SEQ ID NO: 33.

9. The isolated antigen binding protein according to claim 8, wherein the H-FR2 comprises an amino acid sequence shown in SEQ ID NO: 8 or SEQ ID NO: 16.

10. The isolated antigen binding protein according to any one of claims 3-9, which comprises a H-FR3 located between HCDR2 and HCDR3, and the H-FR3 comprises an amino acid sequence shown in SEQ ID NO: 34.

11. The isolated antigen binding protein according to claim 10, wherein the H-FR3 comprises an amino acid sequence shown in SEQ ID NO: 9 or SEQ ID NO: 17.

12. The isolated antigen binding protein according to any one of claims 2-11, comprising a H-FR4, the N-terminus of the H-FR4 is directly or indirectly linked to the C-terminus of the HCDR3, and the H-FR4 comprises an amino acid sequence shown in SEQ ID NO: 35.

13. The isolated antigen binding protein according to claim 12, wherein the H-FR4 comprises an amino acid sequence shown in SEQ ID NO: 10 or SEQ ID NO: 18.

14. The isolated antigen binding protein according to any one of claims 1-13, comprising H-FR1, H-FR2, H-FR3, and H-FR4, the H-FR1 comprises an amino acid sequence shown in SEQ ID NO: 32, the H-FR2 comprises an amino acid sequence shown in SEQ ID NO: 33, the H-FR3 comprises an amino acid sequence shown in SEQ ID NO: 34, and the H-FR4 comprises an amino acid sequence shown in SEQ ID NO: 35.

15. The isolated antigen binding protein according to any one of claims 1-14, comprising H-FR1, H-FR2, H-FR3, and H-FR4, the H-FR1 comprises an amino acid sequence shown in SEQ ID NO: 7 or SEQ ID NO: 15, the H-FR2 comprises an amino acid sequence shown in SEQ ID NO: 8 or SEQ ID NO:16, the H-FR3 comprises an amino acid sequence shown in SEQ ID NO: 9 or SEQ ID NO: 17, and the H-FR4 comprises an amino acid sequence shown in SEQ ID NO: 10 or SEQ ID NO: 18.

16. The isolated antigen binding protein according to any one of claims 1-15, which comprises H-FR1, H-FR2, H-FR3, and H-FR4 selected from any of the following groups:
1) the H-FR1 comprises an amino acid sequence shown in SEQ ID NO: 7, the H-FR2 comprises an amino acid sequence shown in SEQ ID NO: 8, the H-FR3 comprises an amino acid sequence shown in SEQ ID NO: 9, and the H-FR4 comprises an amino acid sequence shown in SEQ ID NO: 10; and
2) the H-FR1 comprises an amino acid sequence shown in SEQ ID NO: 15, the H-FR2 comprises an amino acid sequence shown in SEQ ID NO: 16, the H-FR3 comprises an amino acid sequence shown in SEQ ID NO: 17, and the H-FR4 comprises an amino acid sequence shown in SEQ ID NO: 18;

17. The isolated antigen binding protein according to any one of claims 1-16, which comprises VH, the VH comprises an amino acid sequence shown in SEQ ID NO: 39.

18. The isolated antigen binding protein according to claim 17, wherein the VH comprises an amino acid sequence shown in SEQ ID NO: 22 or SEQ ID NO: 24.

19. The isolated antigen binding protein according to any one of claims 1-18, comprising a heavy chain constant region of an antibody.

20. The isolated antigen binding protein according to claim 19, wherein the heavy chain constant region is derived from a human IgG constant region.

21. The isolated antigen binding protein according to any one of claims 19-20, wherein the heavy chain constant region is derived from a constant region of a human IgG1.

22. The isolated antigen binding protein according to any one of claims 19-21, wherein the heavy chain constant region comprises an amino acid sequence as shown in SEQ ID NO: 26.

23. The isolated antigen binding protein according to any one of claims 1-22, which comprises heavy chain, the heavy chain comprises an amino acid sequence shown in SEQ ID NO: 28 or SEQ ID NO: 30.

24. The isolated antigen binding protein according to any one of claims 1-23, which comprises at least one CDR of the light chain variable region VL of an antibody , the VL comprises an amino acid sequence shown in SEQ ID NO: 40.

25. The isolated antigen binding protein according to any one of claims 1-24, which comprises a LCDR3, the LCDR3 comprises an amino acid sequence shown in SEQ ID NO: 4.

26. The isolated antigen binding protein according to any one of claims 1-25, which comprises a LCDR2, the LCDR2 comprises an amino acid sequence shown in SEQ ID NO: 5.

27. The isolated antigen binding protein according to any one of claims 1-26, which comprises a LCDR1, the LCDR1 comprises an amino acid sequence shown in SEQ ID NO: 6.

28. The isolated antigen binding protein according to any one of claims 1-27, comprising LCDR1, LCDR2, and LCDR3, the LCDR1 comprises an amino acid sequence shown in SEQ ID NO: 6, the LCDR2 comprises an amino acid sequence shown in SEQ ID NO: 5, and the LCDR3 comprises an amino acid sequence shown in SEQ ID NO: 4.

29. The isolated antigen binding protein according to any one of claims 27-28, comprising a L-FR1, wherein the C-terminus of the L-FR1 is directly or indirectly linked to the N-terminus of the LCDR1, and the L-FR1 comprises an amino acid sequence shown in SEQ ID NO: 36.

30. The isolated antigen binding protein according to claim 29, wherein the L-FR1 comprises an amino acid sequence shown in SEQ ID NO: 11 or SEQ ID NO: 19.

31. The isolated antigen binding protein according to any one of claims 27-30, which comprises a L-FR2 located between LCDR1 and LCDR2, and the L-FR2 comprises an amino acid sequence shown in SEQ ID NO: 37.

32. The isolated antigen binding protein according to claim 31, wherein the L-FR2 comprises an amino acid sequence shown in SEQ ID NO: 12 or SEQ ID NO: 20.

33. The isolated antigen binding protein according to any one of claims 26-32, which comprises a L-FR3 located between LCDR2 and LCDR3, and the L-FR3 comprises an amino acid sequence shown in SEQ ID NO: 38.

34. The isolated antigen binding protein according to claim 33, wherein the L-FR3 comprises an amino acid sequence shown in SEQ ID NO: 13 or SEQ ID NO: 21.

35. The isolated antigen binding protein according to any one of claims 25-34, comprising a L-FR4, the N-terminus of the L-FR4 is directly or indirectly linked to the C-terminus of the LCDR3, and the L-FR4 comprises an amino acid sequence shown in SEQ ID NO: 14.

36. The isolated antigen binding protein according to any one of claims 1-35, comprising L-FR1, L-FR2, L-FR3, and L-FR4, the L-FR1 comprises an amino acid sequence shown in SEQ ID NO: 36, the L-FR2 comprises an amino acid sequence shown in SEQ ID NO: 37, the L-FR3 comprises an amino acid sequence shown in SEQ ID NO: 38, and the L-FR4 comprises an amino acid sequence shown in SEQ ID NO: 14.

37. The isolated antigen binding protein according to any one of claims 1-36, comprising L-FR1, L-FR2, L-FR3, and L-FR4, the L-FR1 comprises an amino acid sequence shown in SEQ ID NO: 11 or SEQ ID NO: 19, the L-FR2 comprises an amino acid sequence shown in SEQ ID NO: 12 or SEQ ID NO:20, the L-FR3 comprises an amino acid sequence shown in SEQ ID NO: 13 or SEQ ID NO: 21, and the L-FR4 comprises an amino acid sequence shown in SEQ ID NO: 14.

38. The isolated antigen binding protein according to any one of claims 1-37, which comprises L-FR1, L-FR2, L-FR3, and L-FR4 selected from any of the following groups:
1) the L-FR1 comprises an amino acid sequence shown in SEQ ID NO: 11, the L-FR2 comprises an amino acid sequence shown in SEQ ID NO: 12, the L-FR3 comprises an amino acid sequence shown in SEQ ID NO: 13, and the L-FR4 comprises an amino acid sequence shown in SEQ ID NO: 14; and
2) the L-FR1 comprises an amino acid sequence shown in SEQ ID NO: 19, the L-FR2 comprises an amino acid sequence shown in SEQ ID NO: 20, the L-FR3 comprises an amino acid sequence shown in SEQ ID NO: 21, and the L-FR4 comprises an amino acid sequence shown in SEQ ID NO: 14.

39. The isolated antigen binding protein according to any one of claims 1-38, which comprises VL, the VL comprises an amino acid sequence shown in SEQ ID NO: 40.

40. The isolated antigen binding protein according to claim 39, wherein the VL comprises an amino acid sequence shown in SEQ ID NO: 23 or SEQ ID NO: 25.

41. The isolated antigen binding protein according to any one of claims 1-40, comprising a heavy chain constant region of an antibody.

42. The isolated antigen binding protein according to claim 41, wherein the light chain constant region is derived from a human Igκ constant region.

43. The isolated antigen binding protein according to any one of claims 41-42, wherein the light chain constant region comprises an amino acid sequence as shown in SEQ ID NO: 27.

44. The isolated antigen binding protein according to any one of claims 1-43, which comprises a light chain comprising an amino acid sequence shown in SEQ ID NO: 29 or SEQ ID NO: 31.

45. The isolated antigen binding protein according to any one of claims 1-44, comprising VH and VL selected from any of the following groups:
1) the VH comprises an amino acid sequence shown in SEQ ID NO: 22, and the VL comprises an amino acid sequence shown in SEQ ID NO: 23; and
2) the VH comprises an amino acid sequence shown in SEQ ID NO: 24, and the VL comprises an amino acid sequence shown in SEQ ID NO: 25.

46. The isolated antigen binding protein according to any one of claims 1-45, comprising an antibody or an antigen binding fragment thereof.

47. The isolated antigen binding protein according to any one of claims 1-46, wherein the antigen binding fragment includes Fab, Fab', Fv fragment, F(ab')2, F(ab)2, scFv, di-scFv, and/or dAb.

48. The isolated antigen binding protein according to any one of claims 1-47, wherein the antibody is selected from one or more of the following groups: monoclonal antibodies, chimeric antibodies, humanized antibodies, and fully human antibodies.

49. The isolated antigen binding protein according to any one of claims 1-48, which can specifically bind to pneumolysin protein.

50. The isolated antigen binding protein according to claim 49, wherein the pneumolysin comprises wild-type pneumolysin protein and variants thereof.

51. The isolated antigen binding protein according to any one of claims 49-50, wherein the pneumolysin protein comprises a pneumolysin protein variant in which amino acid residue 146 is deleted compared with the wild-type pneumolysin protein.

52. The isolated antigen binding protein according to any one of claims 1-51, which can prevent and/or treat a disease and/or a condition caused by *Streptococcus pneumoniae.*

53. The isolated antigen binding protein according to claim 52, wherein the diseases and/or conditions include complications of diseases and/or conditions caused by *Streptococcus pneumoniae.*

54. A polypeptide molecule, comprising the isolated antigen binding protein according to any one of claims 1-53.

55. The polypeptide molecule according to claim 54, comprising a fusion protein.

56. An immunoconjugate, comprising the isolated antigen binding protein according to any one of claims 1-53.

57. A nucleic acid molecule encoding the isolated antigen binding protein according to any one of claims 1-53 or the polypeptide molecule according to any one of claims 54-55.

58. A vector comprising the nucleic acid molecule according to claim 57.

59. A cell comprising the nucleic acid molecule according to claim 57 or the vector according to claim 58.

60. A pharmaceutical composition comprising the isolated antigen binding protein according to any one of claims 1-53, the polypeptide molecule according to any one of claims 54-55, the immunoconjugate according to claim 56, the nucleic acid molecule according to claim 57, the vector according to claim 58, and/or the cell according to claim 59, and optionally a pharmaceutically acceptable vehicle.

61. A method for preparing an isolated antigen binding protein according to any one of claims 1-53, wherein the method comprises culturing the cells according to claim 59 under conditions that enable the expression of the antigen binding protein.

62. The isolated antigen binding protein according to any one of claims 1-53, the polypeptide molecule according to any one of claims 54-55, the immunoconjugate according to claim 56, the nucleic acid molecule according to claim 57, the vector according to claim 58, the cell according to claim 59, and/or the pharmaceutical combination according to claim 60, which are used alone or in combination with other drugs.

63. Use of the isolated antigen binding protein according to any one of claims 1-53, the peptide molecule according to any one of claims 54-55, the immunoconjugate according to claim 56, the nucleic acid molecule according to claim 57, the vector according to claim 58, the cell according to claim 59, and/or the pharmaceutical composition according to claim 60 in the manufacture of a medicament for preventing and/or treating a disease and/or condition.

64. The use according to claim 63, wherein the disease and/or condition and complication thereof is caused or mediated by *Streptococcus pneumoniae.*

65. The use according to any one of claims 63-64, wherein the disease and/or condition comprise a complication of disease and/or condition caused or mediated by *Streptococcus pneumoniae.*

66. A method for detecting pneumolysin protein in a sample, comprising administering the isolated antigen binding protein according to any one of claims 1-53, the polypeptide molecule according to any one of claims 54-55, the immunoconjugate according to claim 56, the nucleic acid molecule according to claim 57, the vector according to claim 58, the cell according to claim 59, and/or the pharmaceutical combination according to claim 60.

67. A kit for detecting pneumolysin protein in a sample, comprising the isolated antigen binding protein according to any one of claims 1-53, the polypeptide molecule according to any one of claims 54-55, the immunoconjugate according to claim 56, the nucleic acid molecule according to claim 57, the vector according to claim 58, the cell according to claim 59, and/or the pharmaceutical combination according to claim 60.

68. Use of the isolated antigen binding protein according to any one of claims 1-53, the polypeptide molecule according to any one of claims 54-55, the immunoconjugate according to claim 56, the nucleic acid molecule according to claim 57, the vector according to claim 58, the cell according to claim 59, and/or the pharmaceutical combination according to claim 60 in the manufacture of a kit for detecting the presence and/or content of pneumolysin protein in a sample.
